# EUROPÄISCHE PATENTSCHRIFT

(11) **EP 1 464 658 B1**
(45) Veröffentlichungstag und Bekanntmachung des Hinweises auf die Patenterteilung: **06.10.2010**
(21) Anmeldenummer: 04007371.0
(22) Anmeldetag: 26.03.2004
(51) Int. Cl.: C08F 20/58, C08F 6/12, A61K 8/81

(54) **Verfahren zur Herstellung von stabilen Polymer-Konzentraten**
Process for preparing stable polymer concentrates
Procédé de préparation de concentrés stables de polymères

(30) Priorität: 03.04.2003 DE 10315184
(43) Veröffentlichungstag der Anmeldung: 06.10.2004
(73) Patentinhaber: Clariant Produkte (Deutschland) GmbH, 65929 Frankfurt am Main (DE)
(72) Erfinder: Löffler, Matthias, Dr., 65527 Niedernhausen (DE); Morschhäuser, Roman, Dr., 55122 Mainz (DE)

(56) Entgegenhaltungen:
- WO-A-02/44231

## Beschreibung

Die vorliegende Erfindung betrifft ein Verfahren zur Herstellung von Konzentraten enthaltend Polymere auf Basis von Acryloyldimethyltaurinsäure bzw. deren Salzen, erhältlich durch Polymerisation von Acryloyldimethyltaurinsäure und/oder Acryloyldimethyltauraten in Gegenwart von einer oder mehreren olefinischen Comonomeren, polymeren Additiven und Komponenten mit funktionellen Gruppen.

In der Anmeldung WO 02/44231 wird eine neue Klasse von Polymeren auf Basis von Acryloyldimethyltaurinsäure bzw. deren Salzen beschrieben. Diese Polymere decken breite anwendungstechnische Eigenschaften ab und können als Verdicker, Konsistenzgeber, Emulgator, Dispergator, Gleitmittel, Conditioner und/oder Stabilisator in kosmetischen, dermatologischen und pharmazeutischen Mitteln eingesetzt werden.

Die bevorzugt durch Fällungspolymerisation hergestellten Copolymere auf Basis von Acryloyldimethyltaurinsäure bzw. deren Salzen entsprechend dem Stand der Technik sind pulverförmige Substanzen mit dadurch resultierenden anwendungstechnischen Nachteilen. Pulverförmige Substanzen bergen prinzipiell Staubexplosionsgefahr, ferner ist die Lagerstabilität der Pulver durch Hygroskopie beeinträchtigt.

Zur Verarbeitung bzw. Verwendung der pulverförmigen Produkte ist der Lösevorgang (bevorzugt werden die Polymere in wässrige Medien eingearbeitet) meistens sehr zeitaufwendig. Der Lösevorgang der pulverförmigen Produkte kann, je nach Ansatzgröße, eine Stunde und mehr betragen. Zudem wird häufig eine unvollständige Lösung/Aufquellung der pulverförmigen Produkte beobachtet, was zu einer Qualitäts- und Stabilitätsverminderung der Endformulierung führt (Klumpenund Quaddelbildung). Des weiteren sind bei der Verarbeitung bzw. Verwendung der pulverförmigen Produkte im allgemeinen besondere Rühr- und Dispergiervorrichtungen erforderlich, um die Polymere aus Acryloyldimethyltaurinsäure bzw. deren Salzen in den Mitteln zu lösen, bzw. zu suspendieren.

Ein weiteres Problem ergibt sich bei der Herstellung von hydrophob modifizierten Polymeren mit hohem Anteil an hydrophober Seitenkette. Sobald der Gewichtsanteil der hydrophoben Seitenkette einen kritischen Wert überschreitet (typischerweise > 50 Gew.-%), ist eine Isolierung der resultierenden Polymere auf Grund der amorphen, wachsartigen Konsistenz nur schwer realisierbar. So scheidet eine Fällungspolymerisation auch auf Grund der Löslichkeit in organischen Lösemitteln in diesem Fall aus.

Aufgabe der vorliegenden Erfindung war die Entwicklung eines Eintopfverfahrens zur Herstellung von Polymerkonzentraten, enthaltend Polymere auf Basis von Acryloyldimethyltaurinsäure bzw. deren Salzen in hochkonzentrierter flüssiger oder flüssig-disperser Form mit möglichst hohem Polymeranteil und niedriger Viskosität bei gleichzeitig hoher Stabilität der Lösung bzw. Dispersion in einer kosmetisch und pharmazeutisch akzeptablen Matrix.

Überraschenderweise wurde gefunden, dass lagerstabile und thermostabile Konzentrate aus nachfolgend beschriebenen Polymeren enthaltend Einheiten aus Acryloyldimethyltaurinsäure bzw. deren Salzen hergestellt werden können, indem man im Anschluss an die Polymerisationsreaktion ein Lösungsmittel zusetzt, dessen Siedepunkt höher ist, als der Siedepunkt des zur Polymerisation eingesetzten Polymerisationsmediums bzw. Lösungsmittels und anschließend das niedriger siedende Polymerisationsmedium bzw. Lösungsmittel, gegebenenfalls bei einem Druck, der im Vergleich zu Atmosphärendruck erniedrigt ist, und gegebenenfalls bei einer Temperatur, die im Vergleich zu Raumtemperatur (25°) erhöht ist, entfernt.

Gegenstand der Erfindung ist ein Verfahren zur Herstellung eines Konzentrats in flüssiger oder flüssig-disperser Form, enthaltend
I) 5 bis 80 Gew.-%, bevorzugt 20 bis 60 Gew.-%, besonders bevorzugt 30 bis 40 Gew.-% eines Polymers, erhältlich durch radikalische Polymerisation von Acryloyldimethyltaurinsäure und/oder Acryloyldimethyltauraten [Komponente A)] in Gegenwart einer oder mehrerer Substanzen ausgewählt aus einer oder mehreren der Komponenten D) bis G), und gegebenenfalls zusätzlich in Gegenwart von einer oder mehreren weiteren Substanzen, wobei Komponente
D) aus mindestens monofunktionellen, zur radikalischen Polymerisation befähigten, siliziumhaltigen Substanzen besteht,
E) aus mindestens monofunktionellen, zur radikalischen Polymerisation befähigten, fluorhaltigen Substanzen besteht,
F) aus einfach oder mehrfach olefinisch ungesättigten, gegebenenfalls vernetzenden, Makromonomeren besteht, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um siliziumhaltige Substanzen gemäss Komponente D) oder fluorhaltige Substanzen gemäss Komponente E) handelt, und
G) aus polymeren Additiven mit zahlenmittleren Molekulargewichten von 200 g/mol bis 10⁹ g/mol besteht,
II) 20 bis 95 Gew.-%, bevorzugt 30 bis 80 Gew.-%, besonders bevorzugt 40 bis 60 Gew.-% eines organischen Lösungsmittels oder Lösungsmittelgemisches,
III) 0 bis 60 Gew.-% eines Emulgators, bevorzugt 0 Gew.-% Emulgator für Polymer-Konzentrate mit hohem Anteil an hydrophoben Seitenketten und bevorzugt 5 bis 40 Gew.-%, besonders bevorzugt 10 bis 20 Gew.-% eines Emulgators für Polymer-Konzentrate mit geringem Anteil an hydrophoben Seitenketten und
IV) 0 bis 30 Gew.-%, bevorzugt 0 bis 10 Gew.-%, besonders bevorzugt 0 bis 5 Gew.-%, Wasser,
dadurch gekennzeichnet, dass
a) die Polymerisation von Acryloyldimethyltaurinsäure und/oder Acryloyldimethyltauraten in Gegenwart mindestens einer oder mehrerer Substanzen ausgewählt aus einer oder mehreren der Komponenten D) bis G) und gegebenenfalls in Gegenwart von einer oder mehreren weiteren Substanzen durch eine radikalische Polymerisationsreaktion, vorzugsweise durch Lösungspolymerisation, Gelpolymerisation, nach einem Emulsionsverfahren, Fällungsverfahren, Hochdruckverfahren oder Suspensionsverfahren, in einem Polymerisationsmedium, das sich bezüglich radikalischer Polymerisationsreaktionen weitestgehend inert verhält und die Bildung hoher Molekulargewichte zulässt, vorzugsweise Wasser und niedere, tertiäre Alkohole oder Kohlenwasserstoffe mit 3 bis 30 C-Atomen, besonders bevorzugt t-Butanol, erfolgt,
b) dem aus Schritt a) erhaltenen Gemisch aus Polymer und Polymerisationsmedium ein höhersiedendes Lösungsmittel bzw. Lösungsmittelgemisch, sowie gegebenenfalls Emulgator und/oder Wasser zugesetzt wird, wobei der Siedepunkt des höhersiedenden Lösungsmittels mindestens 10°C höher liegt als der des zur Polymerisation eingesetzten Polymerisationsmediums, und
c) das niedriger siedende Lösungsmittel bzw. Lösungsmittelgemisch, gegebenenfalls bei einem Druck, der im Vergleich zu Atmosphärendruck erniedrigt ist und gegebenenfalls bei einer Temperatur, die im Vergleich zu Raumtemperatur (25°) erhöht ist, entfernt wird.

Bei den Acryloyldimethyltauraten kann es sich um die anorganischen oder organischen Salze der Acryloyldimethyltaurinsäure (Acrylamidopropyl-2-methyl-2-sulfonsäure) handeln. Bevorzugt sind die Li⁺-, Na⁺-, K⁺-, Mg⁺⁺-, Ca⁺⁺-, Al⁺⁺⁺- und/oder NH₄⁺-Salze. Ebenfalls bevorzugt sind die Monoalkylammonium-, Dialkylammonium-, Trialkylammonium- und/oder Tetraalkylammoniumsalze, wobei es sich bei den Alkylsubstituenten der Amine unabhängig voneinander um (C₁-C₂₂)-Alkylreste oder (C₂-C₁₀)-Hydroxyalkylreste handeln kann. Weiterhin sind auch ein- bis dreifach ethoxylierte Ammoniumverbindungen mit unterschiedlichem Ethoxylierungsgrad bevorzugt. Mischungen von zwei- oder mehreren der oben genannten Vertreter sind ebenfalls im Sinne der Erfindung. Der Neutralisationsgrad der Acryloyldimethyltaurinsäure kann zwischen 0 und 100 % betragen, besonders bevorzugt ist ein Neutralisationsgrad von oberhalb 80 %.

Bezogen auf die Gesamtmasse der Polymere beträgt der Gehalt an Acryloyldimethyltaurinsäure bzw. Acryloyldimethyltauraten mindestens 0,1 Gew.-%, vorzugsweise 20 bis 99,5 Gew.-% und besonders bevorzugt 50 bis 98 Gew.-%.

Als polymerisationsfähige, siliziumhaltige Substanzen der Komponente D) sind alle mindestens einfach olefinisch ungesättigten Verbindungen geeignet, die unter den jeweils gewählten Reaktionsbedingungen zur radikalischen Polymerisation befähigt sind. Dabei muss die Verteilung der einzelnen silikonhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen silikonhaltigen Vertretern sind auch möglich. Die Verwendung von silikonhaltigen Substanzen mit zwei oder mehr polymerisationsaktiven Gruppen führt zum Aufbau verzweigter oder vernetzter Strukturen.

Bevorzugte silikonhaltige Substanzen sind solche gemäß Formel (I)

R¹ - Z- [(Si(R³R⁴)-O-)_{w}-(Si(R⁵R⁶)-O)ₓ-Si(R⁸R⁹))- R² (I).

Dabei stellt R¹ einen polymerisationsfähigen Rest aus der Gruppe der vinylisch ungesättigten Verbindungen dar, die zum Aufbau polymerer Strukturen auf radikalischem Wege geeignet ist. Bevorzugt stellt R¹ einen Vinyl-, Allyl-, Methallyl-, Methylvinyl-, Acryl- (CH₂=CH-CO-), Methacryl- (CH₂=C[CH_{3]}-CO-), Crotonyl-, Senecionyl-, Itaconyl-, Maleinyl-, Fumaryl- oder Styrylrest dar.

Zur Anbindung der silikonhaltigen Polymerkette an die reaktive Endgruppe R¹ ist eine geeignete chemische Brücke Z erforderlich. Bevorzugte Brücken Z sind -O-, -O-((C₁-C₅₀)Alkylen)-,-O-((C₆-C₃₀)Arylen)-,-O-((C₅-C₈)Cycloalkylen)-, -O-((C₁-C₅₀)Alkenylen)-, -(Polypropylenoxid)ₙ-, -(Polyethylenoxid)ₒ-, - (Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann. Weiterhin geeignet als Brückengruppierungen Z sind

-O-((C₁ - C₁₀)Alkylen)-(Si(OCH₃)₂)-O- und -O-(Si(OCH₃)₂)-O-.

Der polymere Mittelteil wird durch silikonhaltige Wiederholungseinheiten repräsentiert.

Die Reste R³, R⁴, R⁵ und R⁶ bedeuten unabhängig voneinander -CH₃, -O-CH₃, -C₆H₅ oder -O-C₆H₅.

Die Reste R⁸ und R⁹ bedeuten unabhängig voneinander -CH₃, -O-CH₃, -C₆H₅, -O-C₆H₅ oder -O-Si(CH₃)₃.

Die Indizes w und x repräsentieren stöchiometrische Koeffizienten, die unabhängig voneinander 0 bis 500, bevorzugt 10 bis 250, betragen.

Die Verteilung der Wiederholungseinheiten über die Kette hinweg kann nicht nur rein statistisch, sondern auch blockartig, alternierend oder gradientenartig sein.

R² kann einerseits einen aliphatischen, olefinischen, cycloaliphatischen, arylaliphatischen oder aromatischen (C₁-C₅₀)-Kohlenwasserstoffrest symbolisieren (linear oder verzweigt) oder -OH, -NH₂, -N(CH₃)₂ oder -R⁷ bedeuten oder für die Struktureinheit [-Z-R¹] stehen, wobei Z und R¹ die oben angegebene Bedeutung besitzen. R⁷ steht für weitere Si-haltige Gruppierungen. Bevorzugte R⁷-Reste sind -O-Si(CH₃)₃, -O-Si(Ph)₃, -O-Si(O-Si(CH₃)₃)₂CH₃) und -O-Si(O-Si(Ph)₃)₂Ph). Wenn R² ein Element der Gruppe [-Z-R¹] darstellt, handelt es sich um difunktionelle, Monomere, die zur Vernetzung der entstehenden Polymerstrukturen herangezogen werden können.

Formel (I) beschreibt nicht nur vinylisch funktionalisierte, silikonhaltige Polymerspezies mit einer polymertypischen Verteilung, sondern auch definierte Verbindungen mit diskreten Molekulargewichten.

Besonders bevorzugte silikonhaltige Substanzen sind die folgenden acrylisch- oder methacrylisch modifizierten silikonhaltigen Substanzen:

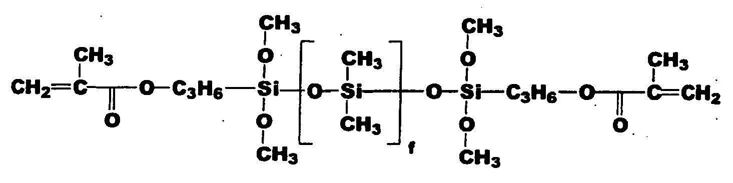

Methacryloxypropyldimethoxysilyl endgeblockte Polydimethylsiloxane (f = 2 bis 500)

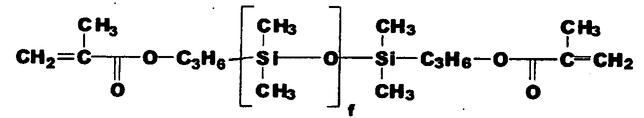

Methacryloxypropyl endgeblockte Polydimethylsiloxane (f = 2 bis 500)

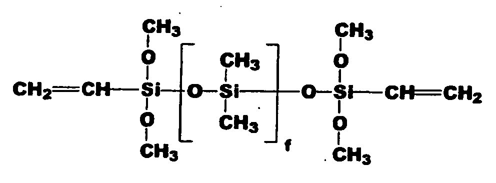

Vinyldimethoxysilyl endgeblockte Polydimethylsiloxane (f = 2 bis 500)

Bezogen auf die Gesamtmasse der Polymere kann der Gehalt an siliziumhaltigen Komponenten bis 99,9 Gew.-%, bevorzugt 0,5 bis 30 Gew.-%, insbesondere bevorzugt 1 bis 20 Gew.-%, betragen.

Als polymerisationsfähige, fluorhaltige Substanzen der Komponente E) sind alle mindestens einfach olefinisch ungesättigten Verbindungen geeignet, die unter den jeweils gewählten Reaktionsbedingungen zur radikalischen Polymerisation befähigt sind. Dabei muss die Verteilung der einzelnen fluorhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen, fluorhaltigen Substanzen der Komponente E) ist auch möglich, wobei dem Experten klar ist, dass monofunktionelle Vertreter zur Bildung kammförmiger Strukturen führen, wohingegen di-, tri-, oder polyfunktionelle Substanzen der Komponente E) zu zumindest teilvernetzten Strukturen führen.

Bevorzugte fluorhaltige Substanzen der Komponente E) sind solche gemäß Formel (II).

R¹-Y-CᵣH₂ᵣCₛF₂ₛCF₃ (II)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ ein Vinyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest dar, besonders bevorzugt einen Acryl- und Methacrylrest.

Zur Anbindung der fluorhaltigen Gruppierung an die reaktive Endgruppe R¹ ist eine geeignete chemische Brücke Y erforderlich. Bevorzugte Brücken Y sind -O-, -C(O)-, - C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃- -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH(CH₃)-CH₂-O]ₙ-[CH₂-CH₂-O]ₘ)ₒ-, wobei n, m und o unabhängig voneinander Zahlen von 0 bis 200 bedeuten und die Verteilung der EO- und PO-Einheiten statistisch oder blockförmig sein kann.

Bei r und s handelt es sich um stöchiometrische Koeffizienten, die unabhängig voneinander Zahlen von 0 bis 200 bedeuten.

Bevorzugte fluorhaltige Substanzen der Komponente E) gemäß Formel (II) sind Perfluorhexylethanol-methacrylat, Perfluorhexoylpropanol-methacrylat, Perfluoroctyethanol-methacrylat, Perfluoroctylpropanol-methacrylat, Perfluorhexylethanolylpolygycolether-methacrylat, Perfluorhexoyl-propanolyl-poly-[ethylglykol-co-propylenglycolether]-acrylat, Perfluoroctyethanolyl-poly-[ethylglykol-blockco-propylenglycolether]-methacrylat, Perfluoroctylpropanolyl-polypropylen-glycolether-methacrylat.

Bezogen auf die Gesamtmasse des Polymeren kann der Gehalt an fluorhaltigen Komponenten bis 99,9 Gew.-%, bevorzugt 0,5 bis 30 Gew.-%, insbesondere bevorzugt 1 bis 20 Gew.-%, betragen.

Bei den Makromonomeren der Komponente F) handelt sich um mindestens einfach olefinisch funktionalisierte Polymere mit einer oder mehreren diskreten Wiederholungseinheiten und einem zahlenmittleren Molekulargewicht größer oder gleich 200 g/mol. Bei der Polymerisation können auch Mischungen chemisch unterschiedlicher Makromonomere der Komponente F) eingesetzt werden. Bei den Makromonomeren handelt es sich um polymere Strukturen, die aus einer oder mehreren Wiederholungseinheit(en) aufgebaut sind und eine für Polymere charakteristische Molekulargewichtsverteilung aufweisen.

Bevorzugt als Makromonomere der Komponente F) sind Verbindungen gemäß Formel (III).

R¹ - Y - [(A)ᵥ - (B)_{w} - (C)ₓ - (D)_{z}] - R² (III)

R¹ stellt eine polymerisationsfähige Funktion aus der Gruppe der vinylisch ungesättigten Verbindungen dar, die zum Aufbau polymerer Strukturen auf radikalischem Wege geeignet sind. Bevorzugt stellt R¹ einen Vinyl-, Allyl-, Methallyl-, Methylvinyl-, Acryl- (CH₂=CH-CO-), Methacryl- (CH₂=C[CH_{3]}-CO-), Crotonyl-, Senecionyl-, Itaconyl-, Maleinyl-, Fumaryl- oder Styrylrest dar.

Zur Anbindung der Polymerkette an die reaktive Endgruppe ist eine geeignete verbrückende Gruppe Y erforderlich. Bevorzugte Brücken Y sind -O-, -C(O)-, - C(O)-O-, -S-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- und -N(CH₃)-, besonders bevorzugt -O-.

Der polymere Mittelteil des Makromonomeren wird durch die diskreten Wiederholungseinheiten A, B, C und D repräsentiert. Bevorzugte Wiederholungseinheiten A, B, C und D leiten sich ab von Acrylamid, Methacrylamid, Ethylenoxid, Propylenoxid, Acryloyldimethyltaurinsäure, Acrylsäure, Methacrylsäure, Methylmethacrylat, Acrylnitril, Maleinsäure, Vinylacetat, Styrol, 1,3-Butadien, Isopren, Isobuten, Diethylacrylamid und Diisopropylacrylamid.

Die Indizes v, w, x und z in Formel (III) repräsentieren die stöchiometrischen Koeffizienten betreffend die Wiederholungseinheiten A, B, C und D. v, w, x, und z betragen unabhängig voneinander 0 bis 500, bevorzugt 1 bis 30, wobei die Summe der vier Koeffizienten im Mittel ≥1 sein muss.

Die Verteilung der Wiederholungseinheiten über die Makromonomerkette kann statistisch, blockartig, alternierend oder gradientenartig sein.

R² bedeutet einen linearen oder verzweigten aliphatischen, olefinischen, cycloaliphatischen, arylaliphatischen oder aromatischen (C₁-C₅₀)-Kohlenwasserstoffrest, OH, -NH₂, -N(CH₃)₂ oder ist gleich der Struktureinheit [-Y-R¹]. Im Falle von R² gleich [-Y-R¹] handelt es sich um difunktionelle Makromonomere, die zur Vernetzung der Copolymere geeignet sind.

Besonders bevorzugt als Makromonomere der Komponente F) sind acrylisch- oder methacrylisch monofunktionalisierte Alkylethoxylate gemäß Formel (IV).

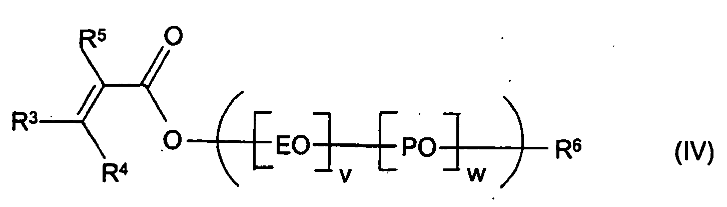

R₃, R₄, R₅ und R₆ bedeuten unabhängig voneinander Wasserstoff oder n-aliphatische, iso-aliphatische, olefinische, cycloaliphatische, arylaliphatische oder aromatische (C₁-C₃₀)-Kohlenwasserstoffreste.

Bevorzugt sind R₃ und R₄ gleich H oder -CH₃, besonders bevorzugt H. R₅ ist vorzugsweise H oder -CH₃. R₆ ist vorzugsweise ein n-aliphatischer, iso-aliphatischer, olefinischer, cycloaliphatischer, arylaliphatischer oder aromatischer (C₁-C₃₀)-Kohlenwasserstoffrest. In einer bevorzugten Ausführungsform ist R⁶ vorzugsweise ein Alkylrest mit 8 bis 24 C-Atomen, insbesondere bevorzugt mit 12 bis 22 C-Atomen.

v und w sind wiederum die stöchiometrischen Koeffizienten betreffend die Ethylenoxideinheiten (EO) und Propylenoxideinheiten (PO). v und w betragen unabhängig voneinander 0 bis 500, bevorzugt 1 bis 30, wobei die Summe aus v und w im Mittel ≥1 sein muss. Die Verteilung der EO- und PO-Einheiten über die Makromonomerkette kann statistisch, blockartig, alternierend oder gradientenartig sein.

Weiterhin insbesondere bevorzugte Makromonomere der Komponente F) haben die folgende Struktur gemäß Formel (IV):

| Bezeichnung | R³ | R⁴ | R⁵ | R⁶ | v | w |
|---|---|---|---|---|---|---|
| Genapol ® LA-030-methacrylat | H | H | -CH₃ | -Lauryl | 3 | 0 |
| Genapol ® LA-070-methacrylat | H | H | -CH₃ | -Lauryl | 7 | 0 |
| Genapol ® LA-200-methacrylat | H | H | -CH₃ | -Lauryl | 20 | 0 |
| Genapol ® LA-250-methacrylat | H | H | -CH₃ | -Lauryl | 25 | 0 |
| Genapol ® T-080-methacrylat | H | H | -CH₃ | -Talk | 8 | 0 |
| Genapol ® T-080-acrylat | H | H | H | -Talk | 8 | 0 |
| Genapol ® T-250-methacrylat | H | H | -CH₃ | -Talk | 25 | 0 |
| Genapol ®T-250-crotonat | -CH₃ | H | -CH₃ | -Talk | 25 | 0 |
| Genapol ®OC-030-methacrylat | H | H | -CH₃ | -Octyl | 3 | 0 |
| Genapol ®OC-105-methacrylat | H | H | -CH₃ | -Octyl | 10 | 5 |
| Genapol ® Behenyl-010-methacrylat | H | H | H | -Behenyl | 10 | 0 |
| Genapol ® Behenyl-020-methacrylat | H | H | H | -Behenyl | 20 | 0 |
| Genapol ® Behenyl-010-senecionyl | -CH₃ | -CH₃ | H | -Behenyl | 10 | 0 |
| Genapol ® PEG-440-diacrylat | H | H | H | -Acryl | 10 | 0 |
| Genapol ® B-11-50-methacrylat | H | H | -CH₃ | -Butyl | 17 | 13 |
| Genapol ® MPEG-750-methacrylat | H | H | -CH₃ | -Methyl | 18 | 0 |
| Genapol ® P-010-acrylat | H | H | H | -Phenyl | 10 | 0 |
| Genapol ® O-050-acrylat | H | H | H | -Oleyl | 5 | 0 |

Weiterhin als Makromonomere der Komponente F) insbesondere geeignet sind Ester der (Meth)acrylsäure mit (C₁₀-C₁₈)-Fettalkoholpolyglykolethern mit 8 EO-Einheiten (Genapol® C-080) C₁₁-Oxoalkoholpolyglykolethern mit 8 EO-Einheiten (Genapol® UD-080) (C₁₂-C₁₄)-Fettalkoholpolyglykolethern mit 7 EO-Einheiten (Genapol® LA-070) (C₁₂-C₁₄)-Fettalkoholpolyglykolethern mit 11 EO-Einheiten (Genapol® LA-110) (C₁₆-C₁₈)-Fettalkoholpolyglykolethern mit 8 EO-Einheiten (Genapol® T-080) (C₁₆-C₁₈)-Fettalkoholpolyglykolethern mit 15 EO-Einheiten (Genapol® T-1 50) (C₁₆-C₁₈)-Fettalkoholpolyglykolethern mit 11 EO-Einheiten (Genapol® T-110) (C₁₆-C₁₈)-Fettalkoholpolyglycolethern mit 20 EO-Einheiten (Genapol® T-200) (C₁₆-C₁₈)-Fettalkoholpolyglycolethern mit 25 EO-Einheiten (Genapol® T-250) (C₁₈-C₂₂)-Fettalkoholpolyglycolethern mit 25 EO-Einheiten und/oder iso-(C₁₆-C₁₈)-Fettalkoholpolyglycolethern mit 25 EO-Einheiten.

Bei den Genapol®-Typen handelt es sich um Produkte der Firma Clariant GmbH.

Bevorzugt beträgt das Molekulargewicht der Makromonomere der Komponente F) 200 g/mol bis 10⁶ g/mol, besonders bevorzugt 150 bis 10⁴ g/mol und insbesondere bevorzugt 200 bis 5000 g/mol.

Bezogen auf die Gesamtmasse der Polymere können geeignete Makromonomere bis zu 99,9 Gew.-% eingesetzt werden. Bevorzugt finden die Bereiche 0,5 bis 30 Gew.-% und 70 bis 99,5 Gew.-% Anwendung. Besonders bevorzugt sind Anteile von 1 bis 20 Gew.-% und 75 bis 95 Gew.-%.

Wenn Acryloydimethyltaurinsäure und/oder Acryloyldimethyltaurate in Gegenwart von weiteren zur radikalischen Polymerisation befähigten Monomeren polymerisiert werden, resultieren Copolymere.

In einer bevorzugten Ausführungsform wird die Polymerisation in Gegenwart mindestens eines polymeren Additivs der Komponente G) durchgeführt, wobei das Additiv der Komponente G) vor der eigentlichen Polymerisation dem Polymerisationsmedium ganz- oder teilweise gelöst zugegeben wird. Die Verwendung von mehreren Additiven der Komponente G) ist ebenfalls erfindungsgemäß. Vernetzte Additive der Komponente G) können ebenfalls verwendet werden.

Die Additive der Komponente G) bzw. deren Mischungen müssen lediglich ganz oder teilweise im gewählten Polymerisationsmedium löslich sein. Während des eigentlichen Polymerisationsschrittes hat das Additiv der Komponente G) mehrere Funktionen. Einerseits verhindert es im eigentlichen Polymerisationsschritt die Bildung übervernetzter Polymeranteile im sich bildenden Polymerisat und andererseits wird das Additiv der Komponente G) gemäß dem allgemein bekannten Mechanismus der Pfropf-Copolymerisation statistisch von aktiven Radikalen angegriffen. Dies führt dazu, dass je nach Additiv der Komponente G) mehr oder weniger große Anteile davon in die Polymere eingebaut werden. Zudem besitzen geeignete Additive der Komponente G) die Eigenschaft, die Lösungsparameter der sich bildenden Polymere während der radikalischen Polymerisationsreaktion derart zu verändern, dass die mittleren Molekulargewichte zu höheren Werten verschoben werden. Verglichen mit analogen Polymeren, die ohne den Zusatz der Additive der Komponente G) hergestellt wurden, zeigen solche, die unter Zusatz von Additiven der Komponente G) hergestellt wurden, vorteilhafterweise eine signifikant höhere Viskosität in wässriger Lösung.

Bevorzugt als Additive der Komponente G) sind in Wasser und/oder Alkoholen, bevorzugt in t-Butanol, lösliche Homo- und Copolymere. Unter Copolymeren sind dabei auch solche mit mehr als zwei verschiedenen Monomertypen zu verstehen.

Besonders bevorzugt als Additive der Komponente G) sind Homo- und Copolymere aus N-Vinylformamid, N-Vinylacetamid, N-Vinylpyrrolidon, Ethylenoxid, Propylenoxid, Acryloyldimethyltaurinsäure, N-Vinylcaprolactam, N-Vinylmethyl-acetamid, Acrylamid, Acrylsäure, Methacrylsäure, N-Vinylmorpholid, Hydroxyethylmethacrylat, Diallyldimethylammoniumchlorid (DADMAC) und/oder [2-(Methacryloyloxy)ethyl]-trimethylammoniumchlorid (MAPTAC); Polyalkylenglykole und/oder Alkylpolyglykole.

Insbesondere bevorzugt als Additive der Komponente G) sind Polyvinylpyrrolidone (z.B. Luviskol K15®, K20® und K30® von BASF), Poly(N-Vinylformamide), Poly(N-Vinylcaprolactame) und Copolymere aus N-Vinylpyrrolidon, N-Vinylformamid und/oder Acrylsäure, die auch teilweise oder vollständig verseift sein können.

Das Molekulargewicht der Additive der Komponente G) beträgt bevorzugt 10² bis 10⁷ g/mol, besonders bevorzugt 0,5^{*}10⁴ bis 10⁶ g/mol.

Die Einsatzmenge des polymeren Additivs der Komponente G) beträgt, bezogen auf die Gesamtmasse der bei der Polymerisation zu polymerisierenden Monomere, bevorzugt 0,1 bis 90 Gew.-%, besonders bevorzugt 1 bis 20 Gew.-% und insbesondere bevorzugt 1,5 bis 10 Gew.-%.

Bei den weiteren Substanzen, die neben Acryloyldimethyltaurinsäure und/oder Acryloyldimethyltauraten und den Substanzen ausgewählt aus einer oder mehreren der Komponenten D) bis G) bei der radikalischen Polymerisation zugegen sind, handelt es sich vorzugsweise um weitere mindestens monofunktionelle, zur radikalischen Polymerisation befähigte Comonomere oder polymere Additive.

Diese weiteren Substanzen werden im folgenden als Substanzen ausgewählt aus Komponente BC) bezeichnet.

In einer bevorzugten Ausführungsform sind die Substanzen der Komponente BC) ausgewählt aus den Comonomeren der Komponente B).

Als Comonomere der Komponente B) können alle olefinisch ungesättigten, nicht kationischen Monomere eingesetzt werden, deren Reaktionsparameter eine Copolymerisation mit Acryloyldimethyltaurinsäure und/oder Acryloyldimethyltauraten in den jeweiligen Reaktionsmedien erlauben.

In einer besonders bevorzugten Ausführungsform sind die Comonomere der Komponente B) aus olefinisch ungesättigten, nicht kationischen, gegebenenfalls vernetzenden, Comonomeren, die wenigstens ein Sauerstoff-, Stickstoff-, Schwefeloder Phosphoratom aufweisen und ein Molekulargewicht kleiner 500 g/mol besitzen, ausgewählt.

Als Comonomere der Komponente B) bevorzugt sind ungesättigte Carbonsäuren und deren Anhydride und Salze, sowie deren Ester mit aliphatischen, olefinischen, cycloaliphatischen, arylaliphatischen oder aromatischen Alkoholen mit einer Kohlenstoffzahl von 1 bis 30.

Als ungesättigte Carbonsäuren besonders bevorzugt sind Acrylsäure, Methacrylsäure, Styrolsulfonsäure, Maleinsäure, Fumarsäure, Crotonsäure, Itaconsäure und Seneciosäure.

Als Gegenionen bevorzugt sind Li⁺, Na⁺, K⁺, Mg⁺⁺, Ca⁺⁺, Al⁺⁺⁺, NH₄⁺, Monoalkylammonium-, Dialkylammonium-, Trialkylammonium- und/oder Tetraalkylammoniumreste, wobei es sich bei den Alkylsubstituenten der Amine unabhängig voneinander um (C₁-C₂₂)-Alkylreste oder (C₂-C₁₀)-Hydroxyalkylreste handeln kann.

Zusätzlich können auch ein- bis dreifach ethoxylierte Ammoniumverbindungen mit unterschiedlichem Ethoxylierungsgrad Anwendung finden. Der Neutralisationsgrad der Carbonsäuren kann zwischen 0 und 100 % betragen.

Als Comonomere der Komponente B) weiterhin bevorzugt sind offenkettige N-Vinylamide, bevorzugt N-Vinylformamid (VIFA), N-Vinylmethylformamid, N-Vinylmethylacetamid (VIMA) und N-Vinylacetamid; cyclische N-Vinylamide (N-Vinyllactame) mit einer Ringgröße von 3 bis 9, bevorzugt N-Vinylpyrrolidon (NVP) und N-Vinylcaprolactam; Amide der Acryl- und Methacrylsäure, bevorzugt Acrylamid, Methacrylamid, N,N-Dimethylacrylamid, N,N-Diethylacrylamid und N,N-Diisopropylacrylamid; alkoxylierte Acryl- und Methacrylamide, bevorzugt Hydroxyethylmethacrylat, Hydroxymethylmethacrylamid, Hydroxyethylmethacrylamid, Hydroxypropylmethacrylamid und Bernsteinsäuremono-[2-(methacryloyloxy)-ethylester]; N,N-Dimethylaminoethylmethacrylat; Diethylaminomethylmethacrylat; Acryl- und Methacrylamidoglykolsäure; 2- und 4-Vinylpyridin; Vinylacetat; Methacrylsäureglycidylester; Styrol; Acrylnitril; Vinylchlorid; Stearylacrylat; Laurylmethacrylat; Vinylidenchlorid; und/oder Tetrafluorethylen.

Als Comonomere der Komponente B) ebenfalls geeignet sind anorganische Säuren und deren Salze und Ester. Bevorzugte Säuren sind Vinylphosphonsäure, Vinylsulfonsäure, Allylphosphonsäure und Methallylsulfonsäure.

Der Gewichtsanteil an Comonomeren der Komponente B), bezogen auf die Gesamtmasse der Polymere, kann 0 bis 99,8 Gew.-% betragen und beträgt bevorzugt 0,5 bis 80 Gew.-%, besonders bevorzugt 2 bis 50 Gew.-%.

In einer weiteren bevorzugten Ausführungsform sind die Substanzen der Komponente BC) ausgewählt aus den Comonomeren der Komponente C).

Als Comonomere der Komponente C) kommen alle olefinisch ungesättigten Monomere mit kationischer Ladung in Frage, die in der Lage sind, in den gewählten Reaktionsmedien mit Acryloyldimethyltaurinsäure oder deren Salzen Copolymere zu bilden. Die dabei resultierende Verteilung der kationischen Ladungen über die Ketten hinweg kann statistisch, alternierend, block- oder gradientenartig sein. Unter den kationischen Comonomeren der Komponente C) sind auch solche zu verstehen, die die kationische Ladung in Form einer betainischen, zwitterionischen, oder amphoteren Struktur tragen.

In einer besonders bevorzugten Ausführungsform sind die Comonomere der Komponente C) aus olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen, ausgewählt.

Comonomere der Komponente C) im Sinne der Erfindung sind auch aminofunktionalisierte Precursor, die durch polymeranaloge Reaktionen in ihre entsprechenden quaternären (z.B. Reaktion mit Dimethylsulfat, Methylchlorid), zwitterionischen (z.B. Reaktion mit Wasserstoffperoxid), betainischen (z.B. Reaktion mit Chloressigsäure), oder amphoteren Derivate überführt werden können.

Besonders bevorzugt als Comonomere der Komponente C) sind Diallyldimethylammoniumchlorid (DADMAC), [2-(Methacryloyloxy)ethyl]trimethylammoniumchlorid (MAPTAC), [2-(Acryloyloxy)ethyl]trimethylammoniumchlorid, [2-Methacrylamidoethyl]trimethylammoniumchlorid, [2-(Acrylamido)ethyl]trimethylammoniumchlorid, N-Methyl-2-vinylpyridiniumchlorid N-Methyl-4-vinylpyridiniumchlorid Dimethylaminoethylmethacrylat, Dimethylaminopropylmethacrylamid, Methacryloylethyl-N-oxid und/oder Methacryloylethyl-betain.

Der Gewichtsanteil an Comonomeren der Komponente C) kann, bezogen auf die Gesamtmasse der Polymere, 0,1 bis 99,8 Gew.-%, vorzugsweise 0,5 bis 30 Gew.-% und besonders bevorzugt 1 bis 20 Gew.-% betragen.

In einer weiteren bevorzugten Ausführungsform sind die erfindungsgemäßen Polymere vernetzt, d.h. sie enthalten Comonomere mit mindestens zwei polymerisationsfähigen Vinylgruppen.

Bevorzugte Vernetzer sind Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate.

Insbesondere bevorzugt als Vernetzer ist Trimethylolpropantriacrylat (TMPTA).

Der Gewichtsanteil an vernetzenden Comonomeren, bezogen auf die Gesamtmasse der Polymere, beträgt bevorzugt bis 20 Gew.-%, besonders bevorzugt 0,05 bis 10 Gew.-% und insbesondere bevorzugt 0,1 bis 7 Gew.-%.

Bevorzugt als Polymere sind solche, die durch Copolymerisation von Substanzen mindestens der Komponenten A), C) und D) erhältlich sind.

Weiterhin bevorzugt als Polymere sind solche, die durch Copolymerisation von Substanzen mindestens der Komponenten A), C) und E) erhältlich sind.

Weiterhin bevorzugt als Polymere sind solche, die durch Copolymerisation von Substanzen mindestens der Komponenten A), D) und F) erhältlich sind.

Weiterhin bevorzugt als Polymere sind solche, die durch Copolymerisation von Substanzen mindestens der Komponenten A) und F) erhältlich sind.

Als Polymerisationsmedium können alle organischen oder anorganischen Lösungsmittel dienen, die sich bezüglich radikalischer Polymerisationsreaktionen weitestgehend inert verhalten und vorteilhafterweise die Bildung mittlerer oder hoher Molekulargewichte zulassen. Bevorzugt Verwendung finden Wasser; niedere Alkohole; bevorzugt Methanol, Ethanol, Propanole, iso-, sec.- und t-Butanol, insbesondere bevorzugt t-Butanol; Kohlenwasserstoffe mit 1 bis 30 Kohlenstoffatomen und Mischungen der vorgenannten Verbindungen.

Die Polymerisationsreaktion erfolgt bevorzugt im Temperaturbereich zwischen 0 und 150°C, besonders bevorzugt zwischen 10 und 100°C, sowohl bei Normaldruck als auch unter erhöhtem oder erniedrigtem Druck. Gegebenenfalls kann die Polymerisation auch unter einer Schutzgasatmosphäre, vorzugsweise unter Stickstoff, ausgeführt werden.

Zur Auslösung der Polymerisation können energiereiche elektromagnetische Strahlen, mechanische Energie oder die üblichen chemischen Polymerisationsinitiatoren, wie organische Peroxide, z.B. Benzoylperoxid, tert.-Butylhydroperoxid, Methylethylketonperoxid, Cumolhydroperoxid, Dilauroylperoxid oder Azoinitiatoren, wie z.B. Azodiisobutyronitril (AIBN), verwendet werden. Ebenfalls geeignet sind anorganische Peroxyverbindungen, wie z.B. (NH₄)₂S₂O₈, K₂S₂O₈ oder H₂O₂, gegebenenfalls in Kombination mit Reduktionsmitteln (z.B. Natriumhydrogensulfit, Ascorbinsäure, Eisen(II)-sulfat etc.) oder Redoxsystemen, welche als reduzierende Komponente eine aliphatische oder aromatische Sulfonsäure (z.B. Benzolsulfonsäure, Toluolsulfonsäure etc.) enthalten.

Als Polymerisationsmedium können alle Lösungsmittel dienen, die sich bezüglich radikalischer Polymerisationsreaktionen weitestgehend inert verhalten und die Bildung hoher Molekulargewichte zulassen. Bevorzugt.Verwendung finden Wasser und niedere, tertiäre Alkohole oder Kohlenwasserstoffe mit 3 bis 30 C-Atomen. In einer besonders bevorzugten Ausführungsform wird t-Butanol als Reaktionsmedium verwendet. Mischungen aus zwei- oder mehreren Vertretern der beschriebenen potentiellen Lösungsmitteln sind selbstverständlich ebenfalls erfindungsgemäß. Dies schließt auch Emulsionen von nicht miteinander mischbaren Solventien ein (z.B. Wasser/Kohlenwasserstoffe). Grundsätzlich sind alle Arten der Reaktionsführung geeignet, die zu den erfindungsgemäßen Polymerstrukturen führen (z.B. Lösungspolymerisation, Fällungsverfahren, Suspensionsverfahren).

Bevorzugt eignet sich die Fällungspolymerisation, besonders bevorzugt die Fällungspolymerisation in tert.-Butanol.

Die nachfolgende Auflistung zeigt 69 Copolymere, die vorzugsweise in den erfindungsgemäßen Konzentraten enthalten sind.

Polymere mit hydrophoben Seitenketten, unvernetzt

| Nr. | Zusammensetzung | Herstellverfahren |
|---|---|---|
| 1 | 95 g AMPS® 5 g Genapol T-080-methacrylat | 1 |
| 2 | 90 g AMPS® 10 g Genapol T-080-methacrylat | 1 |
| 3 | 85 g AMPS® 15 g Genapol T-080-methacrylat | 1 |
| 4 | 80 g AMPS® 20 g Genapol T-080-methacrylat | 1 |
| 5 | 70 g AMPS® 30 g Genapol T-080-methacrylat | 1 |
| 6 | 50 g AMPS® 50 g Genapol T-080-methacrylat | 1,2 |
| 7 | 40 g AMPS® 60 g Genapol T-080-methacrylat | 2 |
| 8 | 30 g AMPS® 70 g Genapol T-080-methacrylat | 2 |
| 9 | 20 g AMPS® 80 g Genapol T-080-methacrylat | 2 |
| 10 | 60 g AMPS® 60 g BB10-acrylat | 1 |
| 11 | 80 g AMPS® 20 g BB10-acrylat | 1 |
| 12 | 90 g AMPS® 10 g BB10-methacrylat | 1 |
| 13 | 80 g AMPS® 20 g BB10-methacrylat | 1 |
| 14 | 80 g AMPS® 20 g Genapol LA040-acrylat | 1 |

Polymere mit hydrophoben Seitenketten, vernetzt

| Nr. | Zusammensetzung | Herstellverfahren |
|---|---|---|
| 15 | 80 g AMPS® 20 g Genapol LA040-methacrylat 0,6 g AMA | 1 |
| 16 | 80 g AMPS® 20 g Genapol LA040-methacrylat 0,8 g AMA | 1 |
| 17 | 80 g AMPS® 20 g Genapol LA040-methacrylat 1,0 g AMA | 1 |
| 18 | 628,73 g AMPS® 120,45 g Genapol T-250-acrylat 6,5 g TMPTA | 2 |
| 19 | 60 g AMPS® 40 g BB10-acrylat 1,9 g TMPTA | 1,2 |
| 20 | 80 g AMPS® 20 g BB10-acrylat 1,4 g TMPTA | 1 |
| 21 | 90 g AMPS® 10 g BB10-methacrylat 1,9 g TMPTA | 1 |
| 22 | 80 g AMPS® 20 g BB25-methacrylat 1,9 g TMPTA | 1 |
| 23 | 60 g AMPS® 40 g BB10-acrylat 1,4 g TMPTA | 1 |

Polymere mit hydrophoben Seitenketten, vernetzt, gepfropft

| Nr. | Zusammensetzung | Herstellverfahren |
|---|---|---|
| 24 | 95 g AMPS® 5 g BB10-acrylat, 1,9 g TMPTA, 1 g Poly-NVP | 1 |
| 25 | 90 g AMPS® 10 g BB10-acrylat, 1,9 g TMPTA, 1 g Poly-NVP | 1 |
| 26 | 85 g AMPS® 15 g BB10-acrylat, 1,9 g TMPTA, 1 g Poly-NVP | 1 |
| 27 | 90g AMPS® 10 g BB10-methacrylat, 1,9 g TMPTA, 1 g Poly-NVP | 1 |

Polymere mit siliziumhaltigen Gruppen, unvernetzt

| Nr. | Zusammensetzung | | Herstellverfahren |
|---|---|---|---|
| 28 | 80 g AMPS®, | 20 g Silvet 867 | 1 |
| 29 | 80 g AMPS® , | 50 g Silvet 867 | 1,2 |

Polymere mit siliziumhaltigen Gruppen, vernetzt

| Nr. | Zusammensetzung | | Herstellverfahren |
|---|---|---|---|
| 30 | 80 g AMPS®, 20 g Silvet 867, | 0,5 g MBA | 1 |
| 31 | 80 g AMPS®, 20 g Silvet 867, | 1,0 g MBA | 1 |
| 32 | 60 g AMPS® , 40 g Y-12867, | 0,95 g AMA | 1 |
| 33 | 80 g AMPS® , 20 g Y-12867, | 0,95 g AMA | 1 |
| 34 | 90 g AMPS® , 10 g Y-12867, | 0,95 g AMA | 1 |
| 35 | 60 g AMPS® , 40 g Silvet 7280, | 0,95 g AMA | 1,2 |
| 36 | 80 g AMPS®, 20 g Silvet 7280, | 0,95 g AMA | 1 |
| 37 | 90 g AMPS®, 10 g Silvet 7280, | 0,95 g AMA | 1 |
| 38 | 60 g AMPS® , 40 g Silvet 7608, | 0,95 g AMA | 1,2 |
| 39 | 80 g AMPS® , 20 g Silvet 7608, | 0,95 g AMA | 1 |
| 40 | 90 g AMPS®, 10 g Silvet 7608, | 0,95 g AMA | 1 |

Polymere mit hydrophoben Seitenketten und kationischen Gruppen, unvernetzt

| Nr. | Zusammensetzung | Herstellverfahren |
|---|---|---|
| 41 | 87,5 g AMPS®, 7,5 g Genapol T-110, 5 g DADMAC | 1 |
| 42 | 40 g AMPS®, 10 g Genapol T110, 45 g Methacrylamid | 1 |
| 43 | 55 g AMPS® , 40 g Genapol LA040, 5 g Quat | 1 |
| 44 | 75 g AMPS®, 10 g BB10, 6,7 g Quat | 1 |

Polymere mit hydrophoben Seitenketten und kationischen Gruppen, vernetzt

| Nr. | Zusammensetzung | Herstellverfahren |
|---|---|---|
| 45 | 60 g AMPS®, 20 g Genapol T-80, 10 g Quat, 10 g HEMA | 1 |
| 46 | 75 g AMPS®, 20 g GenapolT-250, 5 g Quat, 1,4 g TMPTA | 1 |
| 47 | 75 g AMPS®, 20 g GenapolT-250, 10 g Quat, 1,4 g TMPTA | 1 |
| 48 | 75 g AMPS® , 20 g GenapolT-250, 20 g Quat, 1,4 g TMPTA | 1 |

Polymere mit fluorhaltigen Gruppen

| Nr. | Zusammensetzung | Herstellverfahren |
|---|---|---|
| 49 | 94 g AMPS® , 2,02 g Fluowet AC 600 | 1 |
| 50 | 80 g AMPS®, 20 g Perfluoroctylpolyethylenglykolmethacrylat | 1 |

Polymere mit fluorhaltigen Gruppen, gepfropft

| Nr. | Zusammensetzung | Herstellverfahren |
|---|---|---|
| 51 | 80 g AMPS© , 10 g Fluowet AC 600, 5 g Poly-NVP | 1 |
| 52 | 70 g AMPS®, 8 g Perfluoroctylethyloxyglycerinmethacrylat, 5 g Poly-NVP | 1 |

Multifunktionelle Polymere

| Nr. | Zusammensetzung | Herstellverfahren |
|---|---|---|
| 53 | 80 g AMPS®, 10 g Genapol LA070, 10 g Silvet 7608, 1,8 g TMPTA | 1 |
| 54 | 70 g AMPS®, 5 g N-Vinylpyrrolidon, 15 g Genapol T-250 methacrylat, 10 g Quat, 10 g Poly-NVP | 1 |
| 55 | 80 g AMPS® , 5 g N-Vinylformamid, 5 g Genapol O-150-methacrylat, 10 g DADMAC, 1,8 g TMPTA, 8 g Poly-N-Vinylformamid | 1 |
| 56 | 70 g AMPS®, 5 g N-Vinylpyrrolidon, 15 g Genapol T-250-methacrylat, 10 g Quat, 10 g Poly-NVP | 1 |
| 57 | 60 g AMPS®, 10 g Genapol-BE-020-methacrylat, 10 g Genapol T-250-acrylat, 20 g Quat, | 1 |
| 58 | 60 g AMPS®, 20 g MPEG-750-methacrylat, 10 g Methacryloxypropyldimethicon, | 1 |
| | 10 g Perfluorooctylpolyethylenglycol-methacrylat, 10 g Poly[N-vinylcaprolacton-co-acrylsäure] (10/90) | |
| 59 | 80 g AMPS® , 5 g N-Vinylformamid, 5 g Genapol O-150-methacrylat, 10 g DADMAC, 1,8 g TMPTA | 1 |
| 60 | 70 g AMPS®, 10 g Genapol T-250-acrylat, 5 g N-Methyl-4-vinylpyridiniumchlorid, 2,5 g Silvet Y-12867, 2,5 g Perfluorhexylpolyethylenglykolmethacrylat, 10 g Polyethylenglykoldimethacrylat, 4 g Poly[N-Vinylcaprolactam] | 1 |
| 61 | 10 g AMPS®, 20 g Acrylamid, 30 g N-2-Vinylpyrrolidon, 20 g Silvet 7608, 10 g Methacryloxypropyl dimethicon, 10 g Fluowet AC 812 | 2 |
| 62 | 60 g AMPS® , 10 g DADMAC, 10 g Quat, 10 g Genapol-LA-250-crotonat, 10 g Methacryloxypropyldimethicon, 7 g Poly[acrylsäure-co-N-vinylformamid] | 1 |
| 63 | 50 g AMPS®, 45 g Silvet 7608, 1,8 g TMPTA, 8 g Poly[N-Vinylformamid] | 1,2 |
| 64 | 20 g AMPS®, 10 g Genapol T 110, 35 g MAA, 30 g HEMA, 5 g DADMAC | 2 |
| 65 | 20 g AMPS® , 80 g BB10, 1,4 g TMPTA | 2 |
| 66 | 75 g AMPS® , 20gBB10, 6,7g Quat, 1,4gTMPTA | 1 |
| 67 | 35 g AMPS®, 60 g Acrylamid, 2 g VIFA, 2,5 g Vinylphosphonsäure, 2 Mol-% Fluowet EA-600 | 1 |
| 68 | 20 g AMPS® , 80 g BB10 methacrylat, | 2 |
| 69 | 5 g AMPS®, 95 g LA 070 methacrylat | 2 |

Die Copolymere Nr. 1 bis Nr. 69 sind gemäß den folgenden Herstellverfahren 1 oder 2 sythetisierbar.

### Verfahren 1:

Dieses Verfahren eignet sich für Polymere, welche in Fällungsverfahren in einem organischen Medium (bevorzugt t-Butanol, wässriges t-Butanol) herstellbar sind. Dabei werden die Monomere im entsprechenden Lösungsmittel vorgelegt, gegebenenfalls neutralisiert und anschließend durch Zugabe eines Initiators polymerisiert. Zur Vervollständigung der Reaktion und zur Minimierung der Restmonomerkonzentration wird die entstehende Polymersuspension ausreichend lange gerührt. Bei niedrig siedenden organischen Medien wird die Nachrührphase in der Siedehitze durchgeführt. Nach erfolgter Reaktion wird das spätere flüssige Medium und alle Hilfsstoffe zur Polymersuspension unter Rühren hinzugegeben und anschließend das niedriger siedende ehemalige Polymerisationsmedium destillativ entfernt. Zur Vervollständigung der Abtrennung und zur schonenderen Aufarbeitung kann dabei im Vakuum gearbeitet werden. Nach erfolgter Abtrennung wird die Polymersuspension solange nachgerührt, gegebenenfalls unter Zufuhr von Wärme und/oder Zugabe weiterer Ingredienzien (z.B. Wasser, Aktivsubstanzen, etc.), bis die gewünschte Konsistenz und Performance erreicht wird.

### Verfahren 2:

Dieses Verfahren eignet sich für Polymere, welche in Lösungsverfahren in einem organischen Medium (bevorzugt t-Butanol, wässriges t-Butanol) herstellbar sind. Dabei werden die Monomere im entsprechenden Lösungsmittel vorgelegt, gegebenenfalls neutralisiert und anschließend durch Zugabe eines Initiators polymerisiert. Zur Vervollständigung der Reaktion und zur Minimierung der Restmonomerkonzentration wird die entstehende Polymerlösung ausreichend lange gerührt. Bei niedrig siedenden organischen Medien wird die Nachrührphase in der Siedehitze durchgeführt. Nach erfolgter Reaktion wird das spätere flüssige Medium und alle Hilfsstoffe zur Polymerlösung unter Rühren hinzugegeben und anschließend das niedriger siedende ehemalige Polymerisationsmedium destillativ entfernt. Zur Vervollständigung der Abtrennung und zur schonenderen Aufarbeitung kann dabei im Vakuum gearbeitet werden. Nach erfolgter Abtrennung wird die Polymerlösung solange nachgerührt, gegebenenfalls unter Zufuhr von Wärme und/oder Zugabe weiterer Ingredienzien (z.B. Wasser, Aktivsubstanzen, etc.), bis die gewünschte Konsistenz und Performance erreicht wird.

Chemische Bezeichnung der eingesetzten Handelsprodukte:
- AMPS®: Acryloyldimethyltaurat, wahlweise Na- oder NH₄-Salz (AMPS® ist ein Produkt der Firma The Lubrizol Company)
- Genapol® T-080: C₁₆-C₁₈-Fettalkoholpolyglykolether mit 8 EO-Einheiten
- Genapol® T-110: C₁₂-C₁₄-Fettalkoholpolyglykolether mit 11 EO-Einheiten
- Genapol® T-250: C₁₆-C₁₈-Fettalkoholpolyglycolether mit 25 EO-Einheiten
- Genapol® LA-040: C₁₂-C₁₄-Fettalkoholpolyglykolether mit 4 EO-Einheiten
- Genapol® LA-070: C₁₂-C₁₄-Fettalkoholpolyglykolether mit 7 EO-Einheiten
- Genapol® O-150 methacrylat: C₁₆-C₁₈-Fettalkoholpolyglykolether methacrylat mit 15 EO-Einheiten,
- Genapol® LA-250 crotonat: C₁₂-C₁₄-Fettalkoholpolyglykolether crotonat mit 25 EO-Einheiten
- Genapol® T-250 methacrylat: C₁₆-C₁₈-Fettalkoholpolyglycolether methacrylat mit 25 EO-Einheiten
- Genapol® T-250 acrylat: C₁₆-C₁₈-Fettalkoholpolyglycolether methacrylat mit 25 EO-Einheiten
- BB10®: Polyoxyethylen(10)Behenylether
- TMPTA: Trimethylolpropantriacrylat
- Poly-NVP: Poly-N-Vinylpyrrolidon
- Silvet® 867: Siloxan Polyalkylenoxid Copolymer
- MBA: Methylen-bis-acrylamid
- AMA: Allylmethacrylat
- ® Y-12867: Siloxan Polyalkylenoxid Copolymer
- Silvet® 7608: Polyalkylenoxid-modifiziertes Heptamethyltrisiloxan
- Silvet® 7280: Polyalkylenoxid-modifiziertes Heptamethyltrisiloxan
- DADMAC: Diallyldimethyl-ammoniumchlorid
- HEMA: 2-Hydroxyethylmethacrylat
- Quat: 2-(Methacryloyloxy)ethyltrimethylammoniumchlorid
- Fluowet® AC 600: Perfluoralkylethylacrylat
- Span® 80: Sorbitanester

Die beschriebene, optional durchführbare Pfropfung der Copolymere mit anderen Polymeren führt zu Produkten mit besonderer Polymermorphologie, die in wässrigen Systemen optisch klare Gele ergeben. Ein potenzieller Nachteil der Copolymere ohne Pfropfung besteht in einer mehr oder weniger starken Opaleszenz in wässriger Lösung. Diese beruht auf bisher nicht zu vermeidenden, übervernetzten Polymeranteilen, die während der Synthese entstehen und in Wasser nur unzureichend gequollen vorliegen. Dadurch bilden sich Licht streuende Teilchen aus, deren Größe deutlich oberhalb der Wellenlänge des sichtbaren Lichts liegt und deshalb Ursache der Opaleszenz sind. Durch das beschriebene, optional durchführbare Pfropfverfahren wird die Bildung übervernetzter Polymeranteile gegenüber herkömmlichen Techniken deutlich reduziert oder gänzlich vermieden. Die beschriebene, optional durchführbare Inkorporation sowohl von kationischen Ladungen als auch von Silizium-, Fluor- oder Phosphoratomen in die Polymere führt zu Produkten, die in kosmetischen Formulierungen besondere sensorische und rheologische Eigenschaften besitzen. Eine Verbesserung der sensorischen und rheologischen Eigenschaften kann insbesondere bei der Verwendung in rinse off Produkten (insbesondere Haarbehandlungsmittel) als auch leave on Produkten (insbesondere O/W Emulsionen) gewünscht sein.

Die nach dem erfindungsgemäßen Verfahren hergestellten Konzentrate enthalten neben dem Polymer ein organisches Lösungsmittel bzw. Lösungsmittelgemisch, dessen Siedepunkt mindestens 10°C höher liegt als der des eingesetzten Polymerisationsmediums, bevorzugt Öle aus der Gruppe der Kohlenwasserstoffe, Esteröle, pflanzlichen Öle und Silikonöle.

Die erfindungsgemäß eingesetzten Öle umfassen Kohlenwasserstofföle mit linearen oder verzweigten, gesättigten oder ungesättigten C₇-C₄₀-Kohlenstoffketten, beispielsweise Vaseline, Dodecan, Isododecan, Cholesterol, Lanolin, hydrierte Polyisobutylene, Docosane, Hexadecan, Isohexadecan, Paraffine und Isoparaffine; Öle pflanzlichen Ursprungs, insbesondere flüssige Triglyceride wie Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Olivenöl, Erdnussöl, Rapsöl und Kokosnussöl; Öle tierischen Ursprungs, beispielweise Rindertalg, Schweineschmalz, Gänseschmalz, Perhydrosqualen, Lanolin; synthetische Öle wie Purcellinöl, Isoparaffin lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Alkoholester von C₁-C₁₀-Carbonsäuren oder C₂-C₃₀-Dicarbonsäuren, C₁-C₃₀-Carbonsäuremonoester und Polyester von Zucker, C₁-C₃₀-Monoester und Polyester von Glycerin; Wachse wie Bienenwachs, Paraffinwachs oder Mikrowachse, gegebenenfalls in Kombination mit hydrophilen Wachsen, wie z.B. Cetylstearylalkohol; fluorierte und perfluorierte Öle; Monoglyceride von C₁-C₃₀-Carbonsäuren, Diglyceride von C₁-C₃₀-Carbonsäuren, Triglyceride von C₁-C₃₀-Carbonsäuren, beispielsweise Triglyceride der Capryl/Caprinsäuren, Ethylenglykolmonoester von C₁-C₃₀-Carbonsäuren, Ethylenglycoldiester von C₁-C₃₀-Carbonsäuren, Propylenglycolmonoester von C₁-C₃₀-Carbonsäuren, Propylenglycoldiester von C₁-C₃₀-Carbonsäuren, sowie propoxilierte und ethoxilierte Derivate der oben genannten Verbindungsklassen.

An Silikonölen in Betracht kommen Dimethylpolysiloxane, Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, Trimethylsiloxysilicate [(CH₂)₃SiO)_{1/2}]×[SiO₂]_{y}, wobei x für eine Zahl von 1 bis 500 und y für eine Zahl von 1 bis 500 steht, Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoyx-, fluor- und/oder alkylmodifizierte Silikonverbindungen, sowie Polyethersiloxan-Copolymere, wie in US 5,104,645 und den darin zitierten Schriften beschrieben, die bei Raumtemperatur sowohl flüssig als auch harzförmig vorliegen können.

Die nach dem erfindungsgemäßen Verfahren hergestellte Konzentrate können optional Emulgatoren und/oder Wasser enthalten.

Als Emulgatoren kommen in Betracht Anlagerungsprodukte von 0 bis 30 Mol Alkylenoxid, insbesondere Ethylen-, Propylen-, Butylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitanester; (C₁₂-C₁₈)-Fettsäuremonound -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Bevorzugt sind flüssige Fettsäureester, die sowohl ethoxyliert (PEG-10 Polyglyceryl-2 Laurate) als auch nicht ethoxyliert (Polyglyceryl-2 Sesquiisostearate) sein können.

Weitere nach dem erfindungsgemäßen Verfahren hergestellte Konzentrate enthalten bevorzugt Sorbitolester, hergestellt durch Reaktion von Sorbitol mit Fettsäuremethylestern oder Fettsäuretriglyceriden. Der Fettsäurerest in den Fettsäuremethylestern und Fettsäuretriglyceriden enthält im allgemeinen 8 bis 22 C-Atome und kann geradkettig oder verzweigt, gesättigt oder ungesättigt sein. Beispiele hierfür sind Palmitinsäure, Stearinsäure, Laurinsäure, Linolsäure, Linolensäure, Isostearinsäure oder Ölsäure. Als Fettsäuretriglyceride kommen alle nativen tierischen oder pflanzlichen Öle, Fette und Wachse in Frage, beispielsweise Olivenöl, Rapsöl, Palmkernöl, Sonnenblumenöl, Kokosöl, Leinöl, Ricinusöl, Sojabohnenöl, gegebenenfalls auch in raffinierter oder hydrierter Form. Da diese natürlichen Fette, Öle und Wachse normalerweise Mischungen von Fettsäuren mit unterschiedlicher Kettenlänge darstellen, gilt dies auch für die Fettsäurereste in den erfindungsgemäß eingesetzten Sorbitolestern. Die erfindungsgemäß eingesetzten Sorbitolester können auch alkoxyliert sein, vorzugsweise ethoxyliert.

Des weiteren können anionische Emulgatoren, wie ethoxylierte und nicht ethoxylierte mono-, di- oder tri-Phosphorsäureester, aber auch kationische Emulgatoren wie mono-, di- und tri-Alkylquats und deren polymere Derivate eingesetzt werden.

Ebenfalls geeignet sind Gemische von Verbindungen aus mehreren dieser Substanzklassen.

Die nach dem erfindungsgemäßen Verfahren hergestellten Konzentrate eignen sich als Verdicker, Konsistenzgeber, Emulgator, Solubilisator, Dispergator, Gleitmittel, Haftmittel, Conditioner und/oder Stabilisator - hervorragend zur Formulierung von kosmetischen, pharmazeutischen und dermatologischen Mitteln, insbesondere von Öl-in-Wasser Emulsionen in Form von Cremes, Lotionen, Reinigungsmilch, Cremegele, Sprühemulsionen, z.B. Bodylotions, After-Sun Lotions, Sonnenschutzmittel und Deo-Sprays.

Die nach dem erfindungsgemäßen Verfahren hergestellten Konzentrate werden in den kosmetischen und pharmazeutischen Zubereitungen in Gewichtsmengen eingesetzt, dass Polymerkonzentrationen von 0,01 bis 10 Gew.-%, bevorzugt 0,1 bis 5 Gew.-%, besonders bevorzugt 0,5 bis 3 Gew.-%, bezogen auf die fertigen Mittel, resultieren.

Die Mittel können anionische, kationische, nichtionische, zwitterionische und/oder amphotere Tenside, sowie weitere Hilfs- und Zusatzstoffe, kationische Polymere, Filmbildner, Überfettungsmittel, Stabilisatoren, biogene Wirkstoffe, Glycerin, Konservierungsmittel, Perlglanzmittel, Farb- und Duftstoffe, Lösungsmittel, Trübungsmittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen Mitteln antimikrobiell wirkende Agentien zugesetzt werden.

Zusätzlich können die Mittel organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol. Hydrotrop wirken kurzkettige Aniontenside, insbesondere Arylsulfonate, beispielsweise Cumol- oder Toluolsulfonat.

Die nachfolgenden Beispiele sollen den Gegenstand der Erfindung näher erläutern, ohne ihn darauf einzuschränken (bei den Prozentangaben handelt es sich um Gew.-%).

Es wurden verschiedene Basisrezepturen mit hoher Emulgatorkonzentration (A1 bis A6), mit niedriger Emulgatorkonzentration (B1 bis B6) als auch ohne Emulgator (C₁ und C₂) hergestellt. Dabei wurden jeweils verschiedene Polymere aus den obigen Tabellen hergestellt:
Polymer Nr. 1, 4: (niedrig modifiziert, unvernetzt)
Polymer Nr. 17, 18, 22: (niedrig modifiziert, vernetzt)
Polymer Nr. 41, 44: (niedrig modifiziert, kationisch, unvernetzt)
Polymer Nr. 68, 69: (hoch modifiziert, unvernetzt)

### Beispiel A4 Polymerkonzentrat mit hohem Emulgatoranteil

In einem 1 Liter Planschliffkloben mit Temperaturfühler, Rückflusskühler, KPG-Rührer und pH-Kontrolle werden 395 g t-Butanol, 5 g Isopropanol und 80 g Acryloyldimethyltaurinsäure bei 30°C vorgelegt. Anschließend wird durch Einleitung von gasförmigem Ammoniak neutralisiert und die benötigte Menge an Makromonomer (Laureth-7-methacrylat) und 1,5 g Trimethylolpropantriacrylat (Vernetzer) zu der Reaktionsmischung zugegeben. Die Reaktionsmischung wird anschließend durch Einleiten von N₂ inertisiert, auf 60°C aufgeheizt und die Reaktion nach 30 Minuten durch Zugabe von 1 g Dilauroylperoxid gestartet. Es kommt zu einer exothermen Reaktion, bei der die Innentemperatur um mehrere Grad ansteigt. Nach etwa 10 Minuten kommt es zur Ausfällung des entstehenden Polymers, was sich in einem ständigen Anstieg der Lösungsviskosität bemerkbar macht. Nach Beendigung der exothermen Phase (etwa 20-30 Minuten) wird die Reaktionsmischung zur Siedehitze erhitzt und 2 Stunden zur Vervollständigung der Reaktion nachgekocht. Dabei sinkt die Viskosität der Lösung wieder ab. Danach wird der Rückflusskühler durch eine Destillationsbrücke ersetzt. Nun werden 71 g Hostacerin DGI, 105 g Hostaphat KL 340D und 44 g Myrithol 318 zu der Polymersuspension zugegeben und die Hauptmenge an t-Butanol anschließend unter gutem Rühren destillativ entfernt. Durch Anlegen eines Vakuums werden die Reste des Reaktionsbutanols aus der Mischung entfernt. Es ist darauf zu achten, dass das angelegte Vakuum zwar die destillative Abtrennung des t-Butanols ermöglicht, die bei diesem Druck korrespondierende Siedetemperatur des Lösemittels allerdings nicht überschritten wird. Nach erfolgter Abtrennung des t-Butanols wird abgekühlt und das Produkt aus dem Kolben ausgetragen.

Die Polymerkonzentrate A1 bis A3 sowie A5 und A6 werden in analoger Weise hergestellt.

### Beispiel B6 Polymerkonzentrat mit geringem Emulgatoranteil

In einem 1 Liter Planschliffkloben mit Temperaturfühler, Rückflusskühler, KPG-Rührer und pH-Kontrolle werden 400 g t-Butanol und 90 g Acryloyldimethyltaurinsäure bei 30°C vorgelegt. Anschließend wird durch Einleitung von gasförmigem Ammoniak neutralisiert und 15 g N-Vinylpyrrolidon, 4 g Laureth-7-methacrylat und 5 g Beheneth-25-methacrylat zu der Reaktionsmischung zugegeben. Die Reaktionsmischung wird anschließend durch Einleiten von N₂ inertisiert, auf 60°C aufgeheizt und die Reaktion nach 30 Minuten durch Zugabe von 1 g Dilauroylperoxid gestartet. Es kommt zu einer exothermen Reaktion, bei der die Innentemperatur um mehrere Grad ansteigt. Nach etwa 10 Minuten kommt es zur Ausfällung des entstehenden Polymers, was sich in einem ständigen Anstieg der Lösungsviskosität bemerkbar macht. Nach Beendigung der exothermen Phase (etwa 20-30 Minuten) wird die Reaktionsmischung zur Siedehitze erhitzt und 2 Stunden zur Vervollständigung der Reaktion nachgekocht. Dabei sinkt die Viskosität der Lösung wieder ab. Danach wird der Rückflusskühler durch eine Destillationsbrücke ersetzt. Nun werden 8,3 g Hostacerin DGI, 5,5 g Hostaphat KL 340D, 82 g Paraffin und 82 g Isopropylpalmitat zu der Polymersuspension hinzugegeben und anschließend die Hauptmenge an t-Butanol unter gutem Rühren destillativ entfernt. Durch Anlegen eines Vakuums werden die Reste des Reaktionsbutanols aus der Mischung entfernt. Es ist darauf zu achten, dass das angelegte Vakuum zwar die destillative Abtrennung des t-Butanols ermöglicht, die bei diesem Druck korrespondierende Siedetemperatur des Lösemittels allerdings nicht überschritten wird. Nach erfolgter Abtrennung des t-Butanols wird abgekühlt und das Produkt aus dem Kolben ausgetragen.

Die Polymerkonzentrate B1 bis B5 werden in analoger Weise hergestellt

### Beispiel C1 Polymerkonzentrat ohne Emulgator

In einem 1 Liter Planschliffkloben mit Temperaturfühler, Rückflusskühler, KPG-Rührer und pH-Kontrolle werden 400 g t-Butanol und 30 g Acryloyldimethyltaurinsäure bei 30°C vorgelegt. Anschließend wird durch Einleitung von gasförmigem Ammoniak neutralisiert und die benötigte Menge an Makromonomer (70 g Beheneth-25-methacrylat) zu der Reaktionsmischung zugegeben. Die Reaktionsmischung wird anschließend durch Einleiten von N₂ inertisiert, auf 60°C aufgeheizt und die Reaktion nach 30 Minuten durch Zugabe von 1 g Dilauroylperoxid gestartet. Es kommt zu einer exothermen Reaktion, bei der die Innentemperatur um mehrere Grad ansteigt. Nach Beendigung dieser Phase (etwa 20-30 Minuten) wird die Reaktionslösung zur Siedehitze erhitzt und 2 Stunden zur Vervollständigung der Reaktion nachgekocht. Anschließend wird der Rückflusskühler durch eine Destillationsbrücke ersetzt und die Hauptmenge an t-Butanol destillativ entfernt. Nun werden 178g Myrithil 318 (entspricht später 64 Gew.-%) zu dem Polymerkonzentrat hinzugegeben. Dabei löst sich das Polymer in dem Lösungsmittel vollständig auf. Durch Anlegen eines Vakuums werden die Reste des Reaktionsbutanols aus der Mischung entfernt. Es ist darauf zu achten, dass das angelegte Vakuum zwar die destillative Abtrennung des t-Butanols ermöglicht, die bei diesem Druck korrespondierende Siedetemperatur des Lösemittels allerdings nicht überschritten wird. Nach erfolgter Abtrennung des t-Butanols wird abgekühlt und das Produkt aus dem Kolben ausgetragen.

Das Polymerkonzentrat C2 wird in analoger Weise hergestellt.

Die resultierenden Polymer-Konzentrate wurden nach Aussehen, Viskosität sowie Stabilität (Sedimentierung bei Lagerung 25°C, 3 Wochen) beurteilt.

**Tabelle 1: Polymerkonzentrate A1-A6, B1-B6, C1 und C2**

| 1. Hohe Emulgator-konzentration | Polymer Nr. | | | | | |
|---|---|---|---|---|---|---|
| | 18 | 22 | 17 | 41 | 22 | 22 |
| | A1 | A2 | A3 | A4 | A5 | A6 |
| Polymer | 36,0 % | 36,0 % | 36,0 % | 36,0 % | 36,0 % | 36 % |
| Hostacerin DGI | 25,6 % | 12,8 % | 51,2 % | 25,6 % | 28,8 % | 30 % |
| Hostaphat KL 340 D | 6,4 % | 19,2 % | 12,8 % | 38,4 % | 19,2 % | 18 % |
| Myritol 318 | 32,0 % | 32,0 % | 0,0% | 16,0 % | 24,0 % | 16 % |
| Paraffin | 0,0 % | 0,0 % | 0,0 % | 0,0 % | 0,0 % | 0,0 % |
| IPP | 0,0 % | 0,0 % | 0,0 % | 0,0 % | 0,0 % | 0,0 % |
| | | | | | | |

| 2. Niedrige Emulgator-konzentration | Polymer Nr. | | | | | |
|---|---|---|---|---|---|---|
| | 1 | 4 | 44 | 18 | 22 | 22 |
| | B1 | B2 | B3 | B4 | B5 | B6 |
| Polymer | 36,0 % | 36,0% | 36,0 % | 36,0 % | 36,0 % | 36 % |
| Hostacerin DGI | 4,0 % | 2,0 % | 4,0 % | 2,0 % | 3,0 % | 3 % |
| Hostaphat KL 340 D | 1,0 % | 3,0 % | 1,0 % | 3,0 % | 2,0 % | 2 % |
| Myritol 318 | 59,0 % | 59,0 % | 0,0 % | 0,0 % | 29,5 % | 0,0 % |
| Paraffin | 0,0 % | 0,0 % | 59,0 % | 59,0 % | 29,5 % | 29,5 % |
| IPP | 0,0 | 0,0 | 0,0 | 0,0 | 0,0 | 29,5 |

| 3. Ohne Emulgator | Polymer Nr. | | | | | |
|---|---|---|---|---|---|---|
| | 68 | 69 | | | | |
| | C1 | C2 | | | | |
| Polymer | 36,0 % | 36,0% | | | | |
| Hostacerin DGI | 0,0 % | 0,0 % | | | | |
| Hostaphat KL 340 D | 0,0 | 0,0 | | | | |
| Myritol 318 | 64,0 % | 59,0 % | | | | |
| Paraffin | 0,0 % | 5,0 % | | | | |
| IPP | 0,0 | 0,0 | | | | |

### Aussehen:

A1-A6, B1-B6 Dispersionen, gießfähig (Viskosität < 10000 mPas) C1, C2: transparente Lösungen, giessfähig (Viskosität < 10000 mPas) Chemische Bezeichnung der eingesetzten Handelsprodukte:

| | |
|---|---|
| Hostacerin DGI | Polyglyceryl-2 Sesquiisostearate |
| Myritol 318 | Caprylic/Capric Triglyceride |
| IPP | Isopropylpalmitat |
| Hostaphat KL340D | Trilaureth-4-Phosphat |

## Patentansprüche

1. Verfahren zur Herstellung eines Konzentrats in flüssiger oder flüssig-disperser Form, enthaltend
I) 5 bis 80 Gew.-% eines Polymers, erhältlich durch radikalische Polymerisation von Acryloyldimethyltaurinsäure und/oder Acryloyldimethyltauraten [Komponente A)] in Gegenwart einer oder mehrerer Substanzen ausgewählt aus einer oder mehreren der Komponenten D) bis G), und gegebenenfalls zusätzlich in Gegenwart von einer oder mehreren weiteren Substanzen, wobei Komponente
D) aus mindestens monofuriktionellen, zur radikalischen Polymerisation befähigten, siliziumhaltigen Substanzen besteht,
E) aus mindestens monofunktionellen, zur radikalischen Polymerisation befähigten, fluorhaltigen Substanzen besteht,
F) aus einfach oder mehrfach olefinisch ungesättigten, gegebenenfalls vernetzenden, Makromonomeren besteht, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um siliziumhaltige Substanzen gemäss Komponente D) oder fluorhaltige Substanzen gemäss Komponente E) handelt, und
G) aus polymeren Additiven mit zahlenmittleren Molekulargewichten von 200 g/mol bis 10⁹ g/mol besteht,
II) 20 bis 95 Gew.-% eines organischen Lösungsmittels oder Lösungsmittelgemisches,
III) 0 bis 60 Gew.-% eines Emulgators, und
IV) 0 bis 30 Gew.-% Wasser,
**dadurch gekennzeichnet, dass**
a) die Polymerisation von Acryloyldimethyltaurinsäure und/oder Acryloyldimethyltauraten in Gegenwart mindestens einer oder mehrerer Substanzen ausgewählt aus einer oder mehreren der Komponenten D) bis G) und gegebenenfalls zusätzlich in Gegenwart von einer oder mehreren weiteren Substanzen durch eine radikalische Polymerisationsreaktion in einem Polymerisationsmedium, das sich bezüglich radikalischer Polymerisationsreaktionen weitestgehend inert verhält und die Bildung hoher Molekulargewichte zulässt, erfolgt,
b) dem aus Schritt a) erhaltenen Gemisch aus Polymer und Polymerisationsmedium ein höhersiedendes Lösungsmittel bzw. Lösungsmittelgemisch, sowie gegebenenfalls Emulgator und/oder Wasser zugesetzt wird, wobei der Siedepunkt des zugesetzten höhersiedenden Lösungsmittels bzw. Lösungsmittelgemisches mindestens 10°C höher liegt als der des zur Polymerisation eingesetzten Polymerisationsmediums, und
c) das niedriger siedende Polymerisationsmedium, gegebenenfalls bei einem Druck, der im Vergleich zu Atmosphärendruck erniedrigt ist, entfernt wird.

2. Verfahren nach Anspruch 1, **dadurch gekennzeichnet, dass** die weiteren Substanzen ausgewählt sind aus weiteren mindestens monofunktionellen, zur radikalischen Polymerisation befähigten Comonomeren oder polymeren Additiven.

3. Verfahren nach Anspruch 2, **dadurch gekennzeichnet, dass** die weiteren Substanzen zur radikalischen Polymerisation befähigte Comonomere sind und ausgewählt sind aus
a) weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen, und
b) weiteren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen.

4. Verfahren nach einem oder mehreren der Ansprüche 1 bis 3, **dadurch gekennzeichnet, dass** das im Konzentrat enthaltene Polymer 20 bis 99,5 Gew.-%, bezogen auf die Gesamtmasse des Polymers, Acryloyldimethyltaurinsäure und/oder deren Salz enthält.

5. Verfahren nach einem oder mehreren der Ansprüche 1 bis 4, **dadurch gekennzeichnet, dass** das Konzentrat 20 bis 60 Gew.-%, bezogen auf die Gesamtmasse des Konzentrats, an Polymer enthält.

6. Verfahren nach Anspruch 5, **dadurch gekennzeichnet, dass** das Konzentrat 30 bis 40 Gew.-%, bezogen auf die Gesamtmasse des Konzentrats, an Polymer enthält.

7. Verfahren zur Herstellung eines Konzentrates nach einem oder mehreren der Ansprüche 1 bis 6, **dadurch gekennzeichnet, dass** das Konzentrat 30 bis 80 Gew.-%, bezogen auf die Gesamtmasse des Konzentrats, an Emulgator und/oder Lösungsmittel enthält.

8. Verfahren nach Anspruch 7, **dadurch gekennzeichnet, dass** das Konzentrat 40 bis 60 Gew.-%, bezogen auf die Gesamtmasse des Konzentrats, an Emulgator und/oder Lösungsmittel enthält.

9. Verfahren nach einem oder mehreren der Ansprüche 1 bis 8, **dadurch gekennzeichnet, dass** das Konzentrat 0 bis 10 Gew.-%, bezogen auf die Gesamtmasse des Konzentrats, an Wasser enthält.

10. Verfahren nach Anspruch 9, **dadurch gekennzeichnet, dass** das Konzentrat 0 bis 5 Gew.-%, bezogen auf die Gesamtmasse des Konzentrats, an Wasser enthält.

## Claims

1. A process for preparing a concentrate in liquid or liquid-disperse form, comprising
I) from 5 to 80% by weight of a polymer obtainable by free-radically polymerizing acryloyldimethyltaurine and/or acryloyldimethyltaurates [component A)] in the presence of one or more substances selected from one or more of the components D) to G), and optionally additionally in the presence of one or more further substances, component
D) consisting of at least monofunctional silicon-containing substances capable of free-radical polymerization,
E) consisting of at least monofunctional fluorine-containing substances capable of free-radical polymerization,
F) consisting of olefinically mono- or polyunsaturated, optionally crosslinking macromonomers which each have at least one oxygen, nitrogen, sulfur or phosphorus atom and a number-average molecular weight greater than or equal to 200 g/mol, the macromonomers not being silicon-containing substances as per component D) or fluorine-containing substances as per component E), and
G) consisting of polymeric additives having number-average molecular weights of from 200 g/mol to 10⁹ g/mol,
II) from 20 to 95% by weight of an organic solvent or solvent mixture,
III) from 0 to 60% by weight of an emulsifier, and
IV) from 0 to 30% by weight of water,
which comprises
a) effecting the polymerization of acryloyldimethyltaurine and/or acryloyldimethyltaurates in the presence of at least one substance or a plurality of substances selected from one or more of the components D) to G) and optionally additionally in the presence of one or more further substances by a free-radical polymerization reaction in a polymerization medium which behaves very substantially inertly with respect to free-radical polymerization reactions and permits the formation of high molecular weights,
b) adding a higher-boiling solvent or solvent mixture, and also optionally emulsifier and/or water to the mixture of polymer and polymerization medium obtained from step a), the boiling point of the higher-boiling solvent or solvent mixture added being at least 10°C higher than that of the polymerization medium used for the polymerization, and
c) removing the lower-boiling polymerization medium, optionally at a pressure which is reduced compared to atmospheric pressure.

2. The process as claimed in claim 1, wherein the further substances are selected from further at least monofunctional comonomers capable of free-radical polymerization or polymeric additives.

3. The process as claimed in claim 2, wherein the further substances are comonomers capable of free-radical polymerization and are selected from
a) further olefinically unsaturated, noncationic, optionally crosslinking comonomers which have at least one oxygen, nitrogen, sulfur or phosphorus atom and have a molecular weight of less than 500 g/mol, and
b) further olefinically unsaturated, cationic comonomers which have at least one oxygen, nitrogen, sulfur or phosphorus atom and a molecular weight of less than 500 g/mol.

4. The process as claimed in one or more of claims 1 to 3, wherein the polymer present in the concentrate contains from 20 to 99.5% by weight, based on the total mass of the polymer, of acryloyldimethyltaurine and/or its salt.

5. The process as claimed in one or more of claims 1 to 4, wherein the concentrate contains from 20 to 60% by weight, based on the total mass of the concentrate, of polymer.

6. The process as claimed in claim 5, wherein the concentrate contains from 30 to 40% by weight, based on the total mass of the concentrate, of polymer.

7. The process for preparing a concentrate as claimed in one or more of claims 1 to 6, wherein the concentrate contains from 30 to 80% by weight, based on the total mass of the concentrate, of emulsifier and/or solvent.

8. The process as claimed in claim 7, wherein the concentrate contains from 40 to 60% by weight, based on the total mass of the concentrate, of emulsifier and/or solvent.

9. The process as claimed in one or more of claims 1 to 8, wherein the concentrate contains from 0 to 10% by weight, based on the total mass of the concentrate, of water.

10. The process as claimed in claim 9, wherein the concentrate contains from 0 to 5% by weight, based on the total mass of the concentrate, of water.

## Revendications

1. Procédé de préparation d'un concentré sous forme liquide ou en dispersion dans un liquide, contenant
I) 5 à 80 % en poids d'un polymère pouvant être obtenu par polymérisation radicalaire d'acide acryloyldiméthyltaurique et/ou d'acryloyldiméthyltaurates [composant A)] en présence d'une ou plusieurs substances choisies parmi un ou plusieurs des composants D) à G), et éventuellement en outre en présence d'une ou plusieurs substances supplémentaires, le composant D) étant constitué de substances contenant du silicium, au moins monofonctionnelles, aptes à subir une polymérisation radicalaire ; le composant E) étant constitué de substances fluorées, au moins monofonctionnelles, aptes à subir une polymérisation radicalaire ; le composant F) étant constitué de macromonomères comportant une ou plusieurs insaturations oléfiniques, éventuellement réticulables, dont chacun possède au moins un atome d'oxygène, d'azote, de soufre ou de phosphore, et présente une masse moléculaire moyenne supérieure ou égale à 200 g/mol, où, pour ce qui concerne les macromonomères, il ne s'agit pas de substances contenant du silicium selon le composant D) ou de substances fluorées selon le composant E) ; et le composant G) étant constitué d'additifs polymères ayant une masse moléculaire moyenne en nombre de 200 g/mol à 10⁹ g/mol,
II) 20 à 95 % en poids d'un solvant organique ou d'un mélange de solvants organiques,
III) 0 à 60 % en poids d'un émulsifiant, et
IV) 0 à 30 % en poids d'eau,
**caractérisé en ce que**
a) la polymérisation de l'acide acryloyldiméthyltaurique et/ou des acryloyldiméthyltaurates a lieu en présence d'au moins une ou plusieurs substances choisies parmi un ou plusieurs des composants D) à G) et éventuellement en outre en présence d'une ou plusieurs substances supplémentaires, par une réaction de polymérisation radicalaire dans un milieu de polymérisation qui vis-à-vis de réactions de polymérisation radicalaire a un comportement essentiellement inerte, et autorise la formation de masses moléculaires élevées,
b) on ajoute au mélange obtenu dans l'étape a) du polymère et du milieu de polymérisation un solvant ou un mélange de solvants à haut point d'ébullition, ainsi qu'éventuellement un émulsifiant et/ou de l'eau, le point d'ébullition du solvant ou du mélange de solvants à haut point d'ébullition ainsi ajouté étant d'au moins 10°C supérieur à celui du milieu de polymérisation utilisé pour la polymérisation, et
c) on élimine le milieu de polymérisation à bas point d'ébullition, éventuellement sous une pression inférieure à la pression atmosphérique.

2. Procédé selon la revendication 1, **caractérisé en ce que** les substances supplémentaires sont choisies parmi d'autres comonomères ou additifs polymères au moins monofonctionnels, aptes à subir une polymérisation radicalaire.

3. Procédé selon la revendication 2, **caractérisé en ce que** les substances supplémentaires sont des comonomères aptes à subir une polymérisation radicalaire et sont choisies parmi
a) des comonomères supplémentaires à insaturation oléfinique, non cationiques, éventuellement réticulables, qui comprennent au moins un atome d'oxygène, d'azote, de soufre ou de phosphore et ont une masse moléculaire inférieure à 500 g/mol, et
b) des comonomères supplémentaires, cationiques, à insaturation oléfinique, qui comprennent au moins un atome d'oxygène, d'azote, de soufre ou de phosphore et ont une masse moléculaire inférieure à 500 g/mol.

4. Procédé selon l'une ou plusieurs des revendications 1 à 3, **caractérisé en ce que** le polymère contenu dans le concentré contient 20 à 99,5 % en poids, par rapport à la masse totale du polymère, d'acide acryloyldiméthyltaurique et/ou de son sel.

5. Procédé selon l'une ou plusieurs des revendications 1 à 4, **caractérisé en ce que** le concentré contient, par rapport à la masse totale du concentré, 20 à 60 % en poids du polymère.

6. Procédé selon la revendication 5, **caractérisé en ce que** le concentré contient, par rapport à la masse totale du concentré, 30 à 40 % en poids du polymère.

7. Procédé de préparation d'un concentré selon l'une ou plusieurs des revendications 1 à 6, **caractérisé en ce que** le concentré contient 30 à 80 % en poids, par rapport à la masse totale du concentré, d'un émulsifiant et/ou d'un solvant.

8. Procédé selon la revendication 7, **caractérisé en ce que** le concentré contient 40 à 60 % en poids, par rapport à la masse totale du concentré, d'un émulsifiant et/ou d'un solvant.

9. Procédé selon l'une ou plusieurs des revendications 1 à 8, **caractérisé en ce que** le concentré contient, par rapport à la masse totale du concentré, 0 à 10 % en poids d'eau.

10. Procédé selon la revendication 9, **caractérisé en ce que** le concentré contient, par rapport à la masse totale du concentré, 0 à 5 % en poids d'eau.
